Europäisches Patentamt

European Patent Office

Office européen des brevets

(19)

(11) Veröffentlichungsnummer: **0 338 309 B1**

(12) **EUROPÄISCHE PATENTSCHRIFT**

(45) Veröffentlichungstag der Patentschrift: **24.11.93**

(21) Anmeldenummer: **89105798.6**

(22) Anmeldetag: **03.04.89**

(51) Int. Cl.⁵: **C07C 5/25**, C07C 7/163, C07C 2/28, C07C 41/06

(54) **Verfahren zur Isomerisierung von Alkenen mit endständiger Doppelbindung zu Alkenen mit innenständiger Doppelbindung.**

(30) Priorität: **16.04.88 DE 3812683**

(43) Veröffentlichungstag der Anmeldung:
**25.10.89 Patentblatt 89/43**

(45) Bekanntmachung des Hinweises auf die Patenterteilung:
**24.11.93 Patentblatt 93/47**

(84) Benannte Vertragsstaaten:
**DE FR GB IT**

(56) Entgegenhaltungen:
**EP-A- 0 042 537     EP-A- 0 048 893**
**EP-A- 0 087 658     EP-A- 0 197 348**
**BE-A- 735 727       DE-A- 1 800 371**

(73) Patentinhaber: **EC ERDÖLCHEMIE GMBH**
**Alte Strasse 201**
**D-50769 Köln(DE)**

(72) Erfinder: **Schleppinghoff, Bernhard, Dr.**
**Adolf-Kolping-Strasse 5**
**D-4047 Dormagen 1(DE)**
Erfinder: **Köhler, Hans-Dieter, Dr.**
**Stürzelberger Strasse 71**
**D-4047 Dormagen 5(DE)**
Erfinder: **Gabel, Christian, Dr.**
**Goethestrasse 69**
**D-4047 Dormagen 1(DE)**
Erfinder: **Scheef, Hans-Volker**
**Goethestrasse 69**
**D-4047 Dormagen 1(DE)**

(74) Vertreter: **Zobel, Manfred, Dr. et al**
**BAYER AG**
**Konzernverwaltung RP**
**Patente Konzern**
**D-51368 Leverkusen (DE)**

**Beschreibung**

Die vorliegende Erfindung betrifft ein Verfahren zur Isomerisierung von Alkenen mit endständiger Doppelbindung zu solchen mit innenständiger Doppelbindung in Gegenwart von Wasserstoff an einem mit einem hydrieraktiven Metall belegten Kationenaustauscher in der $H^+$-Form.

Geradkettige Alkene unter Einschluß solcher, bei denen eine etwaige Verzweigung nicht an einem C-Atom entspringt, welches die Doppelbindung bildet, haben sich in der Vergangenheit als wertvolle Rohstoffe für verschiedene Reaktionen erwiesen. So werden Alkene mit entständiger Doppelbindung als Monomere oder Comonomere für die Herstellung von Polyolefinen mit wertvollen Eigenschaften geschätzt. Andererseits sind Alkene mit innenständiger Doppelbindung begehrte Alkylierungsmittel zur Alkylierung von n-Alkanen und/oder i-Alkanen, wobei sich wertvolle Motorkraftstoffe ("Alkylatbenzin") ergeben. Bei diesen Alkylierungen sind die Alkene mit innenständiger Doppelbindung gegenüber jenen mit endständiger Doppelbindung bevorzugt, da ihre Alkylate im Treibstoffsektor bessere Eigenschaften aufweisen. So ergibt beispielsweise n-Buten-1 mit n-Butan/i-Butan ein Alkylatbenzin mit einer Research-Octanzahl ROZ von 91, während n-Buten-2 unter gleichen Bedingungen ein Alkylat mit einer ROZ von 97 ergibt.

Als Quelle für Alkene der genannten Art kommen vor allen Dingen die Kohlenwasserstoff-Gemische infrage, die in Crack-Anlagen (beispielsweise Steamcracker oder FCC = Fluid Catalyst Cracker) entstehen. Die in solchen Gemischen aus Crackern vorhandenen Alkene mit endständiger bzw. innenständiger Doppelbindung der gleichen C-Atomzahl stehen nun in einem von den Eigenschaften des jeweiligen Paares an Alkenen abhängigen thermodynamischen Gleichgewicht. Dieses thermodynamische Gleichgewicht liegt bei den hohen Crackertemperaturen stärker auf der Seite des Alkens mit der endständigen Doppelbindung, während es bei tieferen Temperaturen mehr und mehr zu den Alkenen mit innenständiger Doppelbindung verschoben ist.

Es hat daher nicht an Versuchen gefehlt, Alkene mit endständiger Doppelbindung in solche mit innenständiger Doppelbindung katalytisch zu isomerisieren und ihr gegenseitiges Verhältnis dem thermodynamischen Gleichgewicht bei tieferer Temperatur als der Crackertemperatur anzunähern. Eine solche Isomerisierung gelingt z.B. an einem Palladium-Katalysator, wobei in der Literatur wechselnde Angaben über notwendige Hilfsstoffe gemacht worden sind. So wird in FR 78 28 723 berichtet, daß zur Isomerisierung in Gegenwart von Wasserstoff etwas Kohlenmonoxid zugesetzt werden muß. Des weiteren handelt es sich bei den Trägern für solche Palladium-Katalysatoren um hochreine und damit inerte mineralische Stoffe. In DE-OS 31 40 573 wird beispielsweise hochreines $Al_2O_3$ mit einem Gehalt von 0,3 Gew.-% Pd erwähnt, wobei die Neutralisationswärme des Katalysators, gemessen als Neutralisationswärme bei der Ammoniakabsorption, den äußerst kleinen Wert von 6 cal/g zeigt.

Die genannte DE-OS beschreibt darüber hinaus einen zweistufigen Veredelungsprozeß einer $C_4$-Olefin-Fraktion, bei dem in einer ersten Stufe an einem fluorierten Aluminiumoxid, Bor-Aluminiumoxid oder Siliciumdioxid-Aluminiumoxid das in der Olefin-Fraktion enthaltene i-Buten oligomerisiert wird und bei dem in der zweiten Stufe in einem getrennten Katalysatorbett an dem oben beschriebenen Pd-Katalysator n-Buten-1 teilweise zu n-Buten-2 in Anwesenheit von Wasserstoff isomerisiert wird.

In EP 87 658 ist ein Verfahren beschrieben, in welchem bereits ein Kationenaustauscher in der $H^+$-Form mit einem Gehalt an Metallen der VI., VII. und/oder VIII. Nebengruppe des Periodensystems zur katalytischen Umsetzung von i- Alkenen und zur weitgehenden Entfernung von Acetylen- Verbindungen und von Diolefinen, soweit vorhanden, durch katalytische Hydrierung eingesetzt wird. Eine über die zur katalytischen Hydrierung hinausgehende Wasserstoffmenge, die eine Isomerisierung von Alkenen im erfindungsgemäßen Sinn bewirken könnte, ist EP 87 658 nicht zu entnehmen; demzufolge wird eine Isomerisierung der Monoalkene dort nur in untergeordnetem Umfang beobachtet.

Es hat sich nun überraschend gezeigt, daß ein zur genannten Isomerisierung geeigneter Katalysator auch ein nicht-inertes Kationenaustauscherharz in der $H^+$-Form, wie es aus der genannten EP 87 658 bekannt ist, sein kann, obwohl wegen der zahlreichen vorhandenen stark sauren Gruppen unerwünschte Reaktionen, wie Verharzungen und unerwünschte Alkylierungsreaktionen, zu befürchten gewesen wären. Darüber hinaus bietet ein solcher Träger für den Isomerisierungskatalysator weitere Vorteile, die weiter unten näher beschrieben werden.

Die Erfindung betrifft ein Verfahren zur gleichzeitigen Durchführung von

a) der Isomerisierung von Alkenen mit endständiger Doppelbindung zu Alkenen mit innenständiger Doppelbindung und

b) der Selektivhydrierung von hochungesättigten Begleitstoffen in Kohlenwasserstoff-Einsatzmaterialien,

durch Behandlung von Kohlenwasserstoff-Einsatzmaterialien mit einem Gehalt an solchen Alkenen mit endständiger Doppelbindung und einem Gehalt an hochungesättigten Begleitstoffen an einem makroporösen oder gelförmigen Kationenaustauscher in der $H^+$-Form, der 0,001 bis 10 g eines oder mehrerer Metalle der VI. und/oder VII. und/oder VIII. Nebengruppe des Periodensystems der Elemente in elementarer Form pro Liter trockenen Kationenaustauschers enthält und einen Vernetzungsgrad von 2 bis 65 % sowie eine spezifische Oberfläche von 5 bis 750 $m^2/g$ trockenes Austauscherharz aufweist, in Gegenwart von Wasserstoff in der flüssigen Phase, das dadurch gekennzeichnet ist, daß man die Behandlung bei einer Temperatur von 0 bis 120°C durchführt und zusätzlichen Wasserstoff in einer Menge von 100 bis 200 % der stöchiometrisch zur Selektivhydrierung der hochungesättigten Begleitstoffe benötigten Menge einsetzt.

Erfindungsgemäß einzusetzende makroporöse oder gelförmige saure Kationenaustauscher sind dem Fachmann bekannt und können beispielsweise durch Copolymerisation von Vinylmonomeren und Divinylvernetzern, gegebenenfalls in Gegenwart von Lösungsmitteln (DE-AS 1 113 570; US 3.586.646), oder durch Kondensation von Phenol und Formaldehyd hergestellt werden. Vinylmonomere sind beispielsweise Styrol oder Acrylsäureester; ein Divinylvernetzer ist beispielsweise Divinylbenzol. Saure Gruppen solcher Kationenaustauscher sind beispielsweise Carboxylgruppen, Phosphonsäuregruppen oder Sulfonsäuregruppen. Bevorzugt eingesetzt werden stark saure, Sulfonsäuregruppen enthaltende Styrol-Divinylbenzol-Polymerisate, die unter verschiedenen Bezeichnungen handelsüblich sind.

Der Vernetzungsgrad (Menge des Divinylvernetzers, bezogen auf die Gesamtmenge an Comonomeren) beträgt 2 bis 65 %, vorzugsweise 8 bis 25 %. Die spezifische Oberfläche des Kationenaustauschers beträgt 5 bis 750 $m^2/g$, vorzugsweise 50 bis 250 $m^2/g$. Der mittlere Porenradius variiert in den Grenzen von 50 bis 1.200 Å, vorzugsweise 70 bis 500 Å. Solche Kationenaustauscher weisen beispielsweise als Perlpolymerisate Korngrößen von 0,1 bis 2 mm und als Pulverharz Korngrößen von 10 bis 100 $\mu$m auf.

Die Kationenaustauscher werden in der $H^+$-Form eingesetzt, nachdem sie mit 0,001 bis 10 g, bevorzugt 0,2 bis 3 g, bezogen auf 1 l trockenen Kationenaustauscher, eines oder mehrere Metalle der VI. und/oder VII. und/oder VIII. Nebengruppe des Periodensystems der Elemente (Mendelejew) in elementarer Form belegt worden sind. Solche Metalle sind beispielsweise: Chrom, Molybdän, Wolfram, Mangan, Rhenium, Eisen, Kobalt, Nickel, Ruthenium, Rhodium, Palladium, Osmium, Iridium und Platin. Bevorzugt seien genannt: Palladium, Platin, Rhenium, Molybdän oder Nickel; ganz besonders bevorzugt sind Palladium, Platin und Nickel. Auf dem Kationenaustauscher können sich ein oder mehrere der genannten Metalle, bevorzugt jedoch nur eines der genannten Metalle, befinden.

Die Belegung des Kationenaustauschers mit einem oder mehreren der genannten Metalle kann beispielsweise so erfolgen, daß man ein nicht-komplexes, kationisches Salz dieser Metalle mit dem Kationenaustauscher in der $H^+$-Form in an sich bekannter Weise zusammenbringt. Gegebenenfalls wird die hierbei freiwerdende Säure durch geeignete alkalisch reagierende Verbindungen, beispielsweise Alkalihydroxid-Lösungen, neutralisiert. Die Menge des aufzubringenden Metallsalzes wird berechnet oder durch einfache Vorversuche bestimmt, so daß die später in elementarer Form gewünschte Menge an Metall auf den Kationenaustauscher aufgebracht wird.

Der metalldotierte Kationenaustauscher wird neutral gewaschen, getrocknet, beispielsweise bei 80 bis 100°C im Vakuum, und anschließend zur Überführung der aufgebrachten Metalle in den elementaren Zustand mit Wasserstoff gehandelt, beispielsweise bei 2 bis 50 bar, vorzugsweise 20 bis 30 bar, und einer Temperatur von 50 bis 140°C, vorzugsweise 80 bis 120°C. Grundsätzlich können auch andere Reduktionsmittel, wie Hydrazin oder Formaldehyd benutzt werden.

Das erfindungsgemäße Verfahren wird in flüssiger Phase bei einer Temperatur von 0 bis 120°C, bevorzugt 20 bis 90°C, durchgeführt. Hierbei wird ein Druck eingestellt, bei dem das Reaktionsgemisch mit Ausnahme von etwa nicht gelöstem $H_2$ mindestens teilweise flüssig ist. Der Zusammenhang zwischen der gewählten Arbeitstemperatur und einem solchen einzustellenden Druck ist dem Fachmann geläufig. Der metalldotierte Kationenaustauscher liegt hierbei im Festbett oder im Schwebebett vor. Er wird einer Belastung, ausgedrückt durch die LHSV (Liquid Hourly Space Velocity) von 0,1 bis 10, bevorzugt 0,5 bis 8, besonders bevorzugt 1 bis 5, Liter Kohlenwasserstoff-Einsatzmaterial pro Liter Katalysator pro Stunde unterworfen.

In das erfindungsgemäße Verfahren werden Alkene mit 4 bis 7 C-Atomen, bevorzugt mit 4 bis 5 C-Atomen, besonders bevorzugt mit 4 C-Atomen, und einer endständigen Doppelbindung eingesetzt. Solche Alkene sind Buten-1, Penten-1, Hexen-1 und Hepten-1.

Solche Alkene mit endständigen Doppelbindungen können in Raffinerien und petrochemischen Anlagen aus Erdöl-Destillationsschnitten oder bevorzugt aus Destillationsschnitten von Dampfcrackern oder katalytischen Crackern hergestellt werden. Technisch bedeutsam sind weiterhin Kohlenwasserstoff-Einsatzmaterialien mit einem Gehalt an solchen Alkenen mit endständigen Doppelbindungen, wobei zusätzlich beispielsweise geradkettige und verzweigte Alkane im genannten Bereich der C-Atomzahlen von 4 bis 7 oder die zugehörigen Alkene mit innenständigen Doppelbindungen oder beide genannten Verbindungsklassen gemeinsam vorliegen. Während man, ebenso wie bei den obengenannten reinen Alkenen mit endständiger Doppelbindung, grundsätzlich Gemische im genannten Bereich von 4 bis 7 C-Atomen erfindungsgemäß behandeln kann, ist es doch bevorzugt und entspricht der industriellen Praxis, daß breitere oder engere Destillatschnitte innerhalb des genannten Bereiches von 4 bis 7 C-Atomen eingesetzt werden. Solche breiteren oder engeren Destillationsschnitte sind beispielsweise ein $C_5$-$C_7$-Schnitt, ein $C_6$-$C_7$-Schnitt, ein $C_5$-$C_6$-Schnitt, ein $C_4$-$C_5$-Schnitt, in besonders bevorzugter Weise jedoch ein $C_4$-Destillationsschnitt. Typisch für einen $C_4$-Schnitt ist das sogenannte $C_4$-Raffinat II. Dieses entsteht aus dem Roh-$C_4$-Destillationsschnitt der Reaktionsprodukte eines Dampfcrackers oder katalytischen Crackers. Der Roh-$C_4$-Schnitt wird im allgemeinen zunächst einer Extraktion zur Entfernung des Wertstoffes Butadien unterworfen, wobei das sogenannte $C_4$-Raffinat I entsteht. Das $C_4$-Raffinat I wird zur Entfernung von i-Buten einer katalytischen Oligomerisierung oder katalytischen Veretherung dieses i-Butens unterworfen, wobei dann das $C_4$-Raffinat II zurückbleibt. Im $C_4$-Raffinat II schwanken die Gehalte der genannten Stoffe in folgenden Grenzen: n-Butan 15 bis 30 Vol-%, i-Butan 2 bis 8 Vol-%, Buten-1 15 bis 50 Vol-%, Buten-2 20 bis 40 Vol-% und i-Buten 0,1 bis 3 Vol-%.

Wasserstoff kann im erfindungsgemäßen Verfahren in reiner oder technischer Form eingesetzt werden. Wirtschaftlich vorteilhaft kann z.B. ein in petrochemischen Anlagen anfallender, mit Methan und/oder Stickstoff vergesellschafteter Wasserstoff oder ein $H_2$-haltiges Restgas von petrochemischen Anlagen eingesetzt werden.

Der $H_2$-Gehalt in solchen technischen oder reinen Wasserstoffen beträgt 70 bis 100 Vol-% $H_2$, in Restgasen vielfach etwa 80 bis 90 Vol-% $H_2$.

Der Wasserstoff wird bei der erfindungsgemäßen Isomerisierung nicht verbraucht, sondern wirkt katalytisch. Daher ist seine Menge grundsätzlich beliebig, beispielsweise von 5 bis zu 300 Mol-%, bezogen auf den Gehalt an Alken mit endständiger Doppelbindung. Im oberen Teil des genannten Bereiches wird man jedoch bereits mit einer merklichen Hydrierung des für Hydrierungen besonders anfälligen Alkens mit endständiger Doppelbindung rechnen müssen, während das Alken mit innenständiger Doppelbindung etwas stabiler gegen eine solche Hydrierung ist. Im unteren Teil des genannten Bereiches wird man mit geringeren Reaktionsgeschwindigkeiten rechnen müssen, insbesondere wenn im Bereich tieferer Temperaturen und im Bereich höherer LHSV-Werte gearbeitet werden soll. In bevorzugter Weise wird daher in Gegenwart unterstöchiometrischer Mengen Wasserstoff, bezogen auf die Menge des Alkens mit endständiger Doppelbindung gearbeitet werden, beispielsweise im Bereich von 5 bis 95, besonders bevorzugt 10 bis 50, Mol-% $H_2$, bezogen auf das Alken mit endständiger Doppelbindung.

Eine hohe Wasserstoffmenge, auch bis in den Bereich überstöchiometrischer Mengen, kann dann günstig sein, wenn im Bereich hoher LHSV-Werte viel Wasserstoff ungenutzt durch den Isomerisierungsreaktor hindurchfließt.

Im erfindungsgemäßen Verfahren wird das Alken mit endständiger Doppelbindung zu 80 bis 90 %, vielfach bis zu etwa 95 %, des vom thermodynamischen Gleichgewicht zugelassenen Wertes in das Alken mit innenständiger Doppelbindung umgewandelt.

Die Hydrieraktivität des erfindungsgemäß einzusetzenden metalldotierten Kationenaustauschers auf das reaktivere Alken mit endständiger Doppelbindung im Bereich hoher Wasserstoffmengen wurde bereits erwähnt. Diese Hydrieraktivität auf das Alken mit endständiger Doppelbindung sinkt im Bereich kleinerer, insbesondere unterstöchiometrischer Mengen $H_2$ sehr stark ab, so daß in der beschriebenen Weise die erfindungsgemäße Isomerisierung der Alkene ohne wesentliche Hydrierverluste an Alkenen durchgeführt werden kann. Es wurde jedoch beobachtet, daß der erfindungsgemäß einzusetzende metalldotierte Kationenaustauscher auch im Bereich kleiner $H_2$-Mengen eine Hydrieraktivität besitzt, die sich jedoch auf hochungesättigte Begleitstoffe beschränkt und bei diesen eine Selektivhydrierung bis zum Monoalken bewirkt. Als hochungesättigte Begleitstoffe sind hierbei Alkadiene mit isolierten, konjugierten oder kumulierten Doppelbindungen, Alkine und Alkenine zu nennen. Im Bereich von $C_4$-Destillationsschnitten handelt es sich im einzelnen um Butadien-1,3, Butadien-1,2, Butin-1, Butin-2, und Vinyl-acetylen. Weitere hochungesättigte Begleitstoffe in einem $C_4$-Destillationsschnitt können Propin oder Allen sein, also Spuren von nicht vollständig abtrennbaren Anteilen der $C_3$-Kohlenwasserstoffe, wie allgemein alle Destillationsschnitte geringe Spuren von Kohlenwasserstoffen der angrenzenden C-Atomzahlen enthalten. Das erfindungsgemäße Verfahren zur Isomerisierung von Alkenen ist daher mit einer Selektivhydrierung hochungesättigter Begleitstoffe kombiniert. Hierzu wird zusätzlicher Wasserstoff in einer Menge von 100 bis 200 % der stöchiometrisch zur Selektivhydrierung der hochungesättigten Begleitstoffe benötigten Menge eingesetzt.

Diese zur Selektivhydrierung benötigte Menge Wasserstoff ist bei kleinen Anteilen an hochungesättigten Begleitstoffen, bezogen auf die zu isomerisierenden Alkene, im allgemeinen in der für die Isomerisierung benötigten Menge an Wasserstoff bereits enthalten. Die Gesamtwasserstoffmenge kann sodann im Bereich von 10 bis 80 Mol-%, bevorzugt 15 bis 50 Mol-%, bezogen auf die molare Menge an zu isomerisierenden Alken mit endständiger Doppelbindung, begrenzt werden.

4

Neben der Isomerisierungsaktivität und der Hydrieraktivität des erfindungsgemäßen metalldotierten Kationenaustauschers besitzt dieser jedoch noch saure Gruppen in der $H^+$-Form, die in vorteilhafter Weise zur gleichzeitigen Durchführung einer durch solche sauren Gruppen katalysierten Reaktion herangezogen werden können. Eine solche durch saure Gruppen katalysierte Reaktion ist beispielsweise die Oligomerisierung von i-Alkenen.

Diese gleichzeitig verlaufende Oligomerisierung von i-Alkenen ist technisch besonders wichtig im Bereich der $C_4$-Kohlenwasserstoffe; in diesem Falle besteht also das Kohlenwasserstoff-Einsatzmaterial aus einem Gemisch von n-Butan (sowohl 5 bis 10 % aus Dampfcracker bzw. aus katalytischem Cracker), i-Butan (2 bis 5 % bzw. etwa 25 bis 40 %), i-Buten (40 bis 50 % bzw. 12 bis 22 %), Buten-1 (20 bis 30 % bzw. 10 bis 15 %) und Buten-2 (10 bis 25 % bzw. etwa 20 bis 30 %), sowie bis zu 3 % höher ungesättigte Kohlenwasserstoffe. Die Produkte der gleichzeitigen Durchführung der Isomerisierung und Oligomerisierung bestehen dann im Oligomerisat (Diisobutylen, Triisobutylen, Tetraisobutylen und geringe Mengen höherer Oligomerer) und einem davon abtrennbaren $C_4$-Raffinat II, worin der Anteil des ursprünglich vorhandenen Buten-1 zu 90 bis 95 % in Buten-2 umgewandelt ist.

Eine weitere durch saure Gruppen in der $H^+$-Form katalysierte Umwandlung von i-Alkenen ist bekanntlich deren Veretherung mit Alkanolen unter Bildung der zugehörigen Ether, wie Methyl-tert.-butyl-ether (MTBE) tert.-Amylmethylether (TAME) sowie der entsprechenden Ether aus höheren i-Alkenen bzw. unter Einbeziehung anderer Alkanole außer Methanol beispielsweise Ethanol, Propanol, Isopropanol, Butanol oder Isobutanol. Solche Alkanole mit 1 bis 4 C-Atomen werden in einer Menge von 0,7 bis 4 Mol, bevorzugt 0,8 bis 2,5 Mol, besonders bevorzugt 1 bis 2 Mol pro Mol der i-Alkene eingesetzt.

In bevorzugter Weise läßt sich daher das erfindungsgemäße Verfahren der Isomerisierung von Alkenen und mit der Selektivhydrierung hochungesättigter Begleitstoffe auch mit der katalysierten Umsetzung von Isoalkenen, besonders der katalysierten Veretherung von i-Alkenen, kombinieren.

Die Erfindung betrifft daher in bevorzugter Weise weiterhin ein Verfahren zur gleichzeitigen Durchführung von

a) der Isomerisierung von Alkenen mit endständiger Doppelbindung zu Alkenen mit innenständiger Doppelbindung in der oben beschriebenen Weise,
b) der Selektivhydrierung von hochungesättigten Begleitstoffen in Kohlenwasserstoff-Einsatzmaterialien in der ebenfalls oben beschriebenen Weise und
c) der katalysierten Oligomerisierung oder der Veretherung von i-Alkenen in solchen Einsatzmaterialien,

das dadurch gekennzeichnet ist, daß man zur Oligomerisierung von im Kohlenwasserstoff enthaltenen i-Alkenen zusätzlich Wasserstoff in einer Menge von 5 bis zu 300 Mol-%, bezogen auf den Gehalt an Alkenen mit endständiger Doppelbindung, einsetzt oder zur Veretherung der i-Alkene Wasserstoff in einer Menge von 5 bis zu 300 Mol-%, bezogen auf den Gehalt an Alkenen mit endständiger Doppelbindung, und zusätzlich Alkanole mit 1 bis 4 C-Atomen in einer Menge von 0,7 bis 4 Mol pro Mol der i-Alkene einsetzt.

Im vorteilhaften Verfahren unter Einbeziehung der Oligomerisierung oder der Veretherung werden Verfahrensbedingungen im Rahmen der oben beschriebenen Bereiche für die Temperatur, der LHSV und der weiteren genannten Bedingungen angewandt.

Die dem erfindungsgemäßen Verfahren zugrundeliegende erfinderische Idee der Verwendung des oben beschriebenen metalldotierten Kationenaustauschers für die Isomerisierung von Alkenen mit endständiger Doppelbindung zu Alkenen mit innenständiger Doppelbindung und die gleichzeitige Selektivhydrierung betrifft daher in der zuletzt genannten Variante die gleichzeitige Ausnutzung der mehrfunktionellen Aktivität dieses metalldotierten Kationenaustauschers, nämlich der Isomerisierungsaktivität, der Selektivhydrierungsaktivität und der Aktivität zur katalysierten Umsetzung von i-Alkenen.

Beispiel 1

(Herstellung eines erfindungsgemäß einzusetzenden Kationenaustauschers)

Dem handelsüblichen Kationenaustauscher LEWATIT SPC 118 (Styrol-Divinylbenzol-Copolymer mit Sulfonsäuregruppen der Bayer AG, hergestellt nach einem Verfahren entsprechend DE-AS 11 13 570) wurde in der wasserfeuchten $H^+$-Form soviel Palladiumacetat angeboten, daß 1 g Pd pro Liter trockenes Harz nach Reduktion mit $H_2$ auf dem Kationenaustauscher vorhanden war. Die bei der Behandlung mit Palladiumacetat freigesetzte Essigsäure wurde mit 1-gew.-%iger NaOH neutralisiert. Der neutral gewaschene Kationenaustauscher wurde 24 Stunden bei 100 °C im Vakuum einer Wasserstrahlpumpe getrocknet. Das auf dem Kationenaustauscher befindliche Palladium wurde bei 90 bis 100 °C und 20 bis 25 bar $H_2$-Druck innerhalb von 48 Stunden zum Metall reduziert.

Beispiel 2

Oligomerisierung von Raffinat I an einem Pd-dotierten Kationenaustauscher nach Beispiel 1 in Gegenwart von Wasserstoff

In einer Durchlaufapparatur mit zwei hintereinander geschalteten, mantelbeheizten Stahlreaktoren identischer Bauart wurde C4-Raffinat I zu den entsprechenden Oligomeren und einem Buten-2-reichen C4-Raffinat II umgesetzt.

Die Anlage wurde kontinuierlich betrieben, nach 48 Stunden konstanter Fahrweise wurden nach jeder Stufe Analysenproben gezogen, diese wurden gaschromatographisch ausgewertet.

Die Reaktionsführung war folgendermaßen:

| | |
|---|---|
| C4-Raffinat-Einsatz | flüssig |
| Produktflußrichtung | von unten nach oben |
| Wasserstoff | parallel zum Produkt |
| Reaktor 1: Pd-Kationenaustauscher (nach Beispiel 1) | mit Kreislauf (Frischeinsatz/Kreislauf = 1:1) |
| Reaktor 2: Kationenaustauscher (nach Beispiel 1 ohne Pd) | im Durchlauf |
| Stripper | Flüssig/Gasphase |
| Abscheider C4-Abgas-Raffinat | Kondensation der Gasphase |

Folgende Reaktionsbedingungen wurden eingestellt:

| | |
|---|---|
| Reaktionsdruck | 15,0 bar |
| Reaktor 1 LHSV / Temp. | 4,6/20 °C |
| Reaktor 2 LHSV / Temp. | 2,3/75 °C |
| Wasserstoff | 2,0 l/h |
| Molver. Wasserstoff/Butadien | 5,0 |

Die erhaltenen Ergebnisse sind in Tabelle 1 aufgelistet.

Tabelle 1

| Produkt Probestelle | Raffinat I Einsatz | Gesamt-Produkt | | Raffinat II Abgas |
|---|---|---|---|---|
| | | n. Reakt.1 H+/Pd | n. Reakt.2 H+ | |
| Komponente | | | | |
| Gesamt (g/h) | 500.0 | 500.0 | 500.0 | 258.7 |
| Butane (g/h) | 73.0 | 74.5 | 74.5 | 74.5 |
| i-Buten (g/h) | 220.5 | 218.5 | 2.5 | 2.5 |
| Buten-1 (g/h) | 116.0 | 41.5 | 21.8 | 21.8 |
| Buten-2 (g/h) | 89.5 | 163.5 | 157.2 | 157.2 |
| Su-Butene (g/h) | 426.0 | 423.5 | 181.5 | 181.5 |
| Butadien (g/h) | 1,0 | < 0,01 | < 0,01 | < 0,01 |
| Summe C8 (g/h) | < 0,1 | 2,0 | 149,5 | 2,7 |
| Summe C12 (g/h) | < 0,1 | < 0,1 | 81,5 | < 0,1 |
| Summe C16 (g/h) | < 0,1 | < 0,1 | 12,0 | < 0,1 |
| Summe C20 (g/h) | < 0,1 | < 0,1 | 1,0 | < 0,1 |
| Summe Olig (g/h) | < 0,1 | 2,0 | 244,0 | 2,7 |
| Butadien-Hydrierung (%) | | > 99,0 | | |
| Hydrierung der Butene (%) | | 0,7 | | |
| Verhältnis Buten-2:Buten-1 | 0,77 | 3,9 | 7,2 | 7,2 |
| i-C4: Oligomeris. (%) | | 0,9 | 98,9 | |
| n-C4: Oligomeris. (%) | | < 0,1 | 12,7 | |

Beispiel 3

Der Versuch wurde analog zu Beispiel 2 durchgeführt, das Kreislauf-/Frischeinsatz-Verhältnis im Reaktor 1 wurde auf 0,5:1 herabgesetzt.

Zusätzlich wurde die Reaktortemperatur im Reaktor 1 angehoben.

Folgende Reaktionsbedingungen wurden eingestellt:

| | |
|---|---|
| Reaktionsdruck | 15 bar |
| Reaktor 1 LHSV / Temp. | 3,5 / 65 °C |
| Reaktor 2 | 2,3 / 75 °C |
| Wasserstoff | 2,0 l/h |
| Molver. Wasserstoff / Butadien | 5,0 |

Die erhaltenen Ergebnisse sind in Tabelle 2 aufgelistet.

## Tabelle 2

| Produkt | Raffinat I | Gesamt-Produkt | | Raffinat II |
|---|---|---|---|---|
| Probestelle | Einsatz | n.Reakt.1 H+/Pd | n.Reakt.2 H+ | Abgas |
| **Komponente** | | | | |
| Gesamt (g/h) | 500.0 | 500.0 | 500.0 | 254.1 |
| Butane (g/h) | 65.0 | 67.0 | 67.0 | 67.0 |
| i-Buten (g/h) | 226.0 | 90.3 | 3.0 | 3.0 |
| Buten-1 (g/h) | 116.5 | 29.0 | 22.6 | 22.6 |
| Buten-2 | 91.5 | 163.2 | 159.0 | 159.0 |
| Su-Butene | 434.0 | 282.5 | 184.6 | 184.6 |
| Butadien (g/h) | 1.0 | ‹ 0.01 | ‹ 0.01 | ‹ 0.01 |
| Summe C8 (g/h) | ‹ 0.1 | 132.0 | 151.0 | 2.5 |
| Summe C12(g/h) | ‹ 0.1 | 17.0 | 81.0 | ‹ 0.1 |
| Summe C16(g/h) | ‹ 0.1 | 1.0 | 14.9 | ‹ 0.1 |
| Summe C20(g/h) | ‹ 0.1 | 0.5 | 1.5 | ‹ 0.1 |
| Summe Olig (g/h) | ‹ 0.1 | 150.5 | 248.4 | 2.5 |
| Butadien-Hydrierung (%) | | › 99.0 | | |
| Hydrierung der Butene (%) | | 0.7 | | |
| Verhältnis Buten-2 : Buten-1 | 0.8 | 5.6 | 7.0 | |
| i-C4: Oligomeris.(%) | | 60.0 | 98.7 | |
| n-C4: Oligomeris.(%) | | 7.6 | 12.5 | |

Beispiel 4 (Vergleichsbeispiel)

Der Versuch wurde analog zu Beispiel 3 durchgeführt.
Als Katalysator wurde in beiden Reaktoren Kationenaustauscher ohne Pd eingesetzt.
Die Wasserstoffzudosierung entfiel

Tabelle 3

| Produkt Probestelle<br>Komponente | Raffinat I Einsatz | Gesamt n. Reakt.2 H + | Raffinat II Abgas |
|---|---|---|---|
| Gesamt (g/h) | 500.0 | 500.0 | 264.9 |
| Butane (g/h) | 73.0 | 73.0 | 73.0 |
| i-Buten (g/h) | 204.0 | 2.4 | 2.4 |
| Buten-1 (g/h) | 122.5 | 40.2 | 40.2 |
| Buten-2 (g/h) | 99.0 | 146.1 | 146.1 |
| Su.Butene (g/h) | 425.5 | 188.7 | 188.7 |
| Butadien (g/h) | 1.5 | 0.2 | 0.2 |
| Summe C8 (g/h) | < 0.1 | 145.8 | 3.0 |
| Summe C12 (g/h) | < 0.1 | 83.3 | < 0.1 |
| Summe C16 (g/h) | < 0.1 | 8.5 | < 0.1 |
| Summe C20 (g/h) | < 0.1 | 0.5 | < 0.1 |
| Summe Olig(g/h) | < 0.1 | 238.1 | 3.0 |
| Butadien-Dimer. (%) | | 86.7 | |
| Verhältnis Buten-2:Buten-1 | 0.8 | 3.6 | |
| $i\text{-}C_4$ : Oligomeris. (%) | | 98.8 | |
| $n\text{-}C_4$ : Oligomeris. (%) | | 15.9 | |

Beispiel 5:

Der Versuch wurde analog zu Beispiel 2 durchgeführt, anstelle der Oligomerisierung wurde das i-Buten mit Methanol zu MTBE verethert.

Folgende Reaktionsbedingungen wurden eingestellt:

| | |
|---|---|
| Reaktionsdruck | 10,0 bar |
| Reaktor 1 LHSV / Temp. | 2.0 / 65 °C |
| Reaktor 2 LHSV / Temp. | 1.0 / 65 °C |
| Wasserstoff | 3,0 l/h |
| Molver. Wasserstoff/Budadien | 5,0 |

Tabelle 4

| Produkt Probestelle | Raffinat I Einsatz | Gesamt n. Reakt.2 Pd / H + | Raffinat II Abgas |
|---|---|---|---|
| Komponente | | | |
| Gesamt (g/h) | 630.0 | 630.0 | 309.8 |
| Butane (g/h) | 73.0 | 75.0 | 75.0 |
| i-Buten (g/h) | 204.0 | 3.0 | 3.0 |
| Buten-1 (g/h) | 122.5 | 24.5 | 24.5 |
| Buten-2 (g/h) | 99.0 | 199.5 | 196.5 |
| Su.Butene (g/h) | 425.5 | 224.0 | 224.0 |
| Butadien (g/h) | 1.5 | < 0.01 | < 0.01 |
| Methanol (g/h) | 130.0 | 15.2 | 10.8 |
| MTBE (g/h) | < 0.1 | 315.8 | < 0.1 |
| Butadien-Hydrierung (%) | | > 99.0 | |
| Hydrierung der Butene (%) | | 0.2 | |
| Verhältnis Buten-2:Buten-1 | 0.8 | 8.0 | |
| i-C4: Veretherung (%) | | 98.5 | |

Beispiel 6 (Vergleichsbeispiel)

Die Veretherung wurde analog zu Beispiel 3 durchgeführt, das i-Buten wurde mit Methanol zu MTBE verethert.

Es wurde ohne Wasserstoffzugabe gearbeitet.

Folgende Reaktionsbedingungen wurden eingestellt:

| | |
|---|---|
| Reaktionsdruck | 10. bar |
| Reaktor 1 LHSV / Temp. | 2.0 / 65 ° C |
| Reaktor 2 LHSV / Temp. | 1.0 / 65 ° C |

Tabelle 5

| Produkt Probestelle | Raffinat I Einsatz | Gesamt n. Reakt.2 H + | Raffinat II Abgas |
|---|---|---|---|
| Komponente | | | |
| Gesamt (g/h) | 630.0 | 530.0 | 311.4 |
| Butane (g/h) | 73.0 | 73.0 | 73.0 |
| i-Buten (g/h) | 204.0 | 5.1 | 5.0 |
| Buten-1 (g/h) | 122.5 | 120.0 | 88.0 |
| Buten-2 (g/h) | 99.0 | 101.5 | 133.0 |
| Su.Butene (g/h) | 425.5 | 226.6 | 226.0 |
| Butadien (g/h) | 1.5 | 0.4 | 0.4 |
| Methanol (g/h) | 130.0 | 16.3 | 12.0 |
| MTBE (g/h) | < 0.1 | 312.6 | < 0.1 |
| Höhere | | 1.1 | |
| Butadien-Dimer. (%) | | 74.0 | |
| Verhältnis Buten-2:Buten-1 | 0.8 | 0.8 | |
| i-C4 Veretherung (%) | | 97.5 | |

Beispiel 7

Der Versuch wurde analog zu Beispiel 2 durchgeführt.
Der Reaktor 2 wurde umfahren, Butadien wurde hydriert und das Buten-1 zum Buten-2 isomerisiert.
Folgende Reaktionsbedingungen wurden eingestellt:

| Reaktiondruck | 15.0 bar |
|---|---|
| Reaktor 1 LHSV / Temp. | 6.0 / 40 °C |
| Wasserstoff | 2.0 l/h |
| Molver. Wasserstoff / Butadien | 5.0 |

Tabelle 6

| Produkt Probestelle | Raffinat I Einsatz | Gesamt n. Reaktor Pd/H + |
|---|---|---|
| Komponente | | |
| Gesamt (g/h) | 500.0 | 500.0 |
| Butane (g/h) | 73.0 | 75.5 |
| i-Buten (g/h) | 204.0 | 204.0 |
| Buten-1 (g/h) | 122.5 | 28.0 |
| Buten-2 (g/h) | 99.0 | 192.5 |
| Su.Butene (g/h) | 425.5 | 424.5 |
| Butadien (g/h) | 1.5 | 0.01 |
| Butadien-Hydrierung (%) | | > 99.0 |
| Hydrierung der Butene (%) | | 0.5 |
| Verhältnis Buten-2:Buten-1 | 0.8 | 6.9 |

Beispiel 8 (Vergleichsbeispiel)

Der Versuch wurde wie Beispiel 8 durchgeführt.
Als Katalysator wurde der Kationenaustauscher nach Beispiel 1 ohne Pd ohne Wasserstoffzugabe eingesetzt.
Folgende Reaktionsbedingungen wurden eingestellt:

| Reaktiondruck | 15.0 bar |
|---|---|
| Reaktor 1 LHSV / Temp. | 3.0 / 40 °C |

## Tabelle 8

| Produkt<br><br>Probestelle | C5-Schnitt<br><br>Einsatz | Gesamt<br><br>n.Reaktor<br>H+ | |
|---|---|---|---|
| Komponente | | | |
| Gesamt (g/h) | 500.0 | 500.0 | |
| Pentane (g/h) | 166.0 | 166.0 | |
| 3-MB-1 (g/h) | 1.0 | 1.0 | 3-MB-1 (3-Methylbuten-1) |
| 2-MB-1 (g/h) | 13.5 | 13.0 | 2-MB-1 (2-Methylbuten-1) |
| 2-MB-2 (g/h) | 56.0 | 56.5 | 2-MB-2 (1-Methylbuten-1) |
| Su.Methylbu-<br>tene | 70.5 | 70.5 | |
| C5-Diene(g/h) | 3.5 | 1.0 | |
| Höhere (g/h) | < 0.1 | 2.5 | |
| Rest-KW | 260.0 | 260.0 | |
| C5-Dien<br>Oligomeris.<br>(%) | | 71.4 | |
| Verhältnis<br>2MB-2 zu 2MB-1 | 4.1 | 4.3 | |

**Patentansprüche**

1. Verfahren zur gleichzeitigen Durchführung von

   a) der Isomerisierung von Alkenen mit endständiger Doppelbindung zu Alkenen mit innenständiger Doppelbindung und

   b) der Selektivhydrierung von hochungesättigten Begleitstoffen in Kohlenwasserstoff-Einsatzmaterialien,

   durch Behandlung von Kohlenwasserstoff-Einsatzmaterialien mit einem Gehalt an solchen Alkenen mit endständiger Doppelbindung und einem Gehalt an hochungesättigten Begleitstoffen an einem makroporösen oder gelförmigen Kationenaustauscher in der H⁺-Form, der 0,001 bis 10 g eines oder mehrerer Metalle der VI. und/oder VII. und/oder VIII. Nebengruppe des Periodensystems der Elemente in elementarer Form pro Liter trockenen Kationenaustauschers enthält und einen Vernetzungsgrad von 2 bis 65 % sowie eine spezielle Oberfläche von 5 bis 750 m²/g trockenes Austauscherharz aufweist, in Gegenwart von Wasserstoff in der flüssigen Phase, dadurch gekennzeichnet, daß man die Behandlung bei einer Temperatur von 0 bis 120°C durchführt und zusätzlichen Wasserstoff in einer Menge von 100 bis 200 % der stöchiometrisch zur Selektivhydrierung der hochungesättigten Begleitstoffe benötigten Menge einsetzt.

2. Verfahren nach Anspruch 1, dadurch gekennzeichnet, daß man die Behandlung bei 20 bis 90°C durchführt.

**3.** Verfahren nach Ansprüchen 1 und 2, dadurch gekennzeichnet, daß man zur Behandlung eine LHSV (Liquid Hourly Space Velocity) von 0,1 bis 10 l Einsatzmaterial pro Liter Katalysator pro Stunde, bevorzugt LHSV = 0,5 bis 8, besonders bevorzugt LHSV = 1 bis 5, einstellt.

**4.** Verfahren nach Ansprüchen 1 bis 3, dadurch gekennzeichnet, daß das Einsatzmaterial neben den Alkenen mit endständiger Doppelbindung solche mit innenständiger Doppelbindung und/oder Alkane enthält.

**5.** Verfahren nach Anspruch 1, dadurch gekennzeichnet, daß man zur Oligomerisierung von im Kohlenwasserstoff enthaltenen i-Alkenen zusätzlich Wasserstoff in einer Menge von 5 bis zu 300 Mol-%, bezogen auf den Gehalt an Alkenen mit endständiger Doppelbindung, einsetzt oder zur Veretherung der i-Alkene Wasserstoff in einer Menge von 5 bis zu 300 Mol-%, bezogen auf den Gehalt an Alkenen mit endständiger Doppelbindung, und zusätzlich Alkanole mit 1 bis 4 C-Atomen in einer Menge von 0,7 bis 4 Mol pro Mol der i-Alkene einsetzt.

**6.** Verfahren nach Ansprüchen 1 bis 5, dadurch gekennzeichnet, daß die Einsatzmaterialien Kohlenwasserstoffe mit 4 bis 7 C-Atomen, bevorzugt 4 bis 5 C-Atomen, besonders bevorzugt 4 C-Atomen, enthalten.

## Claims

**1.** Process for carrying out at the same time
a) the isomérisation of alkenes having a terminal double bond to give alkenes having an internal double bond, and
b) the selective hydrogenation of highly unsaturated accompanying compounds in hydrocarbon feed materials,
by treating hydrocarbon feed materials containing alkenes of this type having a terminal double bond and containing highly unsaturated accompanying compounds in the presence of hydrogen in the liquid phase on a macroporous or gel-like cation exchanger in the $H^+$ form which contains 0.001 to 10 g of one or more metals of the VIth and/or VIIth and/or VIIIth sub-group of the periodic system of the elements in elementary form per litre of dry cation exchanger and which has a degree of crosslinking of 2 to 65% and a specific surface area of 5 to 750 $m^2$/g of dry exchanger resin, characterised in that the treatment is carried out at a temperature from 0 to 120°C and additional hydrogen is employed in a proportion of 100 to 200% of the proportion required by stoichiometry for the selective hydrogenation of the highly unsaturated accompanying compounds.

**2.** Process according to Claim 1, characterised in that the treatment is carried out at 20 to 90°C.

**3.** Process according to Claims 1 and 2, characterised in that an LHSV (liquid hourly space velocity) of 0.1 to 10 l of feed material per litre of catalyst and per hour, preferably an LHSV of 0.5 to 8 and particularly preferably an LHSV of 1 to 5, is established for the treatment.

**4.** Process according to Claims 1 to 3, characterised in that, in addition to the alkenes having a terminal double bond, the feed material contains alkenes having an internal double bond and/or alkanes.

**5.** Process according to to Claim 1, characterised in that, for the oligomerisation of isoalkenes contained in the hydrocarbon, hydrogen is additionally employed in a proportion of 5 up to 300 mol %, based on the content of alkenes having a terminal double bond, or, for the etherification of the isoalkenes, hydrogen in a proportion of 5 up to 300 mol %, based on the content of alkenes having a terminal double bond, and, in addition, alkanols having 1 to 4 C atoms in a proportion of 0.7 to 4 mol per mole of the isoalkenes are employed.

**6.** Process according to Claims 1 to 5, characterised in that the feed materials contain hydrocarbons having 4 to 7 C atoms, preferably 4 to 5 C atoms and particularly preferably 4 C atoms.

## Revendications

**1.** Procédé de conduite simultanée
a) de l'isomérisation d'alcènes à double liaison terminale en alcènes à double liaison interne et

b) de l'hydrogénation sélective d'impuretés fortement insaturées dans des charges hydrocarbonées, par traitement des charges hydrocarbonées contenant de tels alcènes à double liaison terminale et des impuretés fortement insaturées, sur un échangeur cationique à pores macroscopiques ou gélifié sous la forme H$^+$, qui contient 0,001 à 10 g d'un ou plusieurs métaux des sous-groupes VI et/ou VII et/ou VIII du Système Périodique des Eléments sous la forme élémentaire, par litre d'échangeur cationique sec et qui présente un degré de réticulation de 2 à 65 % de même qu'une surface spécifique de 5 à 750 m$^2$/g de résine échangeuse sèche, en phase liquide en présence d'hydrogène, caractérisé en ce qu'on conduit le traitement à une température de 0 à 120°C et on utilise de l'hydrogène en supplément en une quantité de 100 à 200 % de la quantité nécessaire stoechiométriquement pour l'hydrogénation sélective des impuretés fortement insaturées.

2. Procédé suivant la revendication 1, caractérisé en ce qu'on conduit le traitement à 20-90°C.

3. Procédé suivant les revendications 1 et 2, caractérisé en ce qu'on règle pour le traitement une LHSV (Liquid Hourly Space Velocity) de 0,1 à 10 litres de charge par litre de catalyseur par heure, de préférence une LHSV de 0,5 à 8 et notamment une LHSV de 1 à 5.

4. Procédé suivant les revendications 1 à 3, caractérisé en ce que la charge contient, à côté des alcènes à double liaison terminale, des alcènes à double liaison interne et/ou des alcanes.

5. Procédé suivant la revendication 1, caractérisé en ce qu'on utilise en outre pour l'oligomérisation d'iso-alcènes contenus dans l'hydrocarbure, de l'hydrogène en une quantité de 5 à 300 moles % par rapport à la teneur en alcènes à double liaison terminale ou bien on utilise pour l'éthérification des iso-alcènes de l'hydrogène en une quantité de 5 à 300 moles % par rapport à la teneur en alcènes à double liaison terminale, et en outre des alcanols ayant 1 à 4 atomes de carbone en une quantité de 0,7 à 4 moles par mole des iso-alcènes.

6. Procédé suivant les revendications 1 à 5, caractérisé en ce que les charges contiennent des hydrocarbures ayant 4 à 7 atomes de carbone, de préférence 4 à 5 atomes de carbone, notamment 4 atomes de carbone.